# EUROPEAN PATENT APPLICATION

(11) **EP 0 725 136 A1**
(43) Date of publication of application: **07.08.1996**
(21) Application number: 95400239.0
(22) Date of filing: 03.02.1995
(51) Int. Cl.: C12N 15/12, C12N 15/63, C12Q 1/68, C07K 14/47, C07K 16/18, G01N 33/53, A61K 48/00, C12N 5/10, A01K 67/00

(54) **Nucleotide and peptide sequences of the USHER-lB gene diagnostic and therapeutic applications**

(71) Applicant: INSTITUT PASTEUR, F-75724 Paris Cédex 15 (FR); IMPERIAL COLLEGE OF SCIENCE TECHNOLOGY AND MEDICINE, London SW7 2AZ (GB); MEDICAL RESEARCH COUNCIL, London W1N 4AL (GB)
(72) Inventor: Petit, Christine, F-92350 Le Plessis Robinson (FR); Weil, Dominique, F-75015 Paris (FR); Blanchard, Stephane, F-94550 Chevilly Larue (FR); Rown, Steve D.M, London N4 3SD (GB); Steel, Karen P., Nottingham NG7 2RD (GB)
(74) Representative: Le Guen, Gérard

(57) **Abstract**

The invention relates to DNA comprising all or part of a nucleotide sequence selected from SEQ ID N° 1, sequences hybridizing to all or part of SEQ ID N° 1 and any sequence which code for the same aminoacid sequence as encoded by the nucleotide sequence SEQ ID N° 1 due to the degeneracy of the genetic code, as well as to a method of selection of a genetic abnormality linked to a syndromic or non-syndromic deafness and a pharmaceutical composition comprising all or part of the nucleic acid of a gene encoding an unconventional myosin molecule.

SEQUENCE LISTING

(1) GENERAL INFORMATION:

(i) APPLICANT:
(A) NAME: institut pasteur
(B) STREET: 28 rue du docteur roux
(C) CITY: paris
(E) COUNTRY: france
(F) POSTAL CODE (ZIP): 75724
(G) TELEPHONE: 45 68 80 93
(H) TELEFAX: 40 61 30 17
(I) TELEX: 250 609

(A) NAME: imperial college of science technology and
medicine
(B) STREET: sherfield building
(C) CITY: LONDON
(E) COUNTRY: GREAT BRITAIN
(F) POSTAL CODE (ZIP): SW7 2AZ

(A) NAME: MEDICAL RESEARCH COUNCIL
(B) STREET: 20 PARK CRESCENT
(C) CITY: LONDON
(E) COUNTRY: GREAT BRITAIN
(F) POSTAL CODE (ZIP): WIN 4AL

(ii) TITLE OF INVENTION: NUCLEOTIDE AND PEPTIDE SEQUENCES OF THE
USHER_1B GENE, DIAGNOSTIC AND THERAPEUTIC APPLICATIONS

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release £1.0, Version £1.30 (EPO)

(v) CURRENT APPLICATION DATA:
APPLICATION NUMBER: EP 95400239.0

(2) INFORMATION FOR SEQ ID NO: 1:

(i) SEQUENCE CHARACTERISTICS:
(A) LENGTH: 1405 base pairs
(B) TYPE: nucleic acid
(C) STRANDEDNESS: double
(D) TOPOLOGY: linear

(ii) MOLECULE TYPE: cDNA

(iv) ANTI-SENSE: NO

(xi) SEQUENCE DESCRIPTION: SEQ ID NO: 1:

ACGTATACGG GCTCCATCCT GGTGGCTGTG AACCCCTACC AGCTGCTCTC CATCTACTCG 60

CCAGAGCACA TCCGCCAGTA TACCAACAAG AAGATTGGGG AGATGCCCCC CCACATCTTT 120

GCCATTGCTG ACAACTGCTA CTTCAACATG AAACGCAACA GCCGAGACCA GTGCTGCATC 180

ATCAGTGGGG AATCTGGGGC CGGGAAGACG GAGAGCACAA AGCTGATCCT GCAGTTCCTG 240

GCAGCCATCA GTGGGCAGCA CTCGTGGATT GAGCAGCAGG TCTTGGAGGC CACCCCCATT 300

CTGGAAGCAT TTGGGAATGC CAAGACCATC CGCAATGACA ACTCAAGCCG TTTCGGAAAG 360

TACATCGACA TCCACTTCAA CAAGCGGGGC GCCATCGAGG GCGCGAAGAT TGAGCAGTAC 420

CTGCTGGAAA AGTCACGTGT CTGTCGCCAG GCCCTGGATG AAAGGAACTA CCACGTGTTC 480

TACTGCATGC TGGAGGGTAT GAGTGAGGAT CAGAAGAAGA AGCTGGGCTT GGGCCAGGCC 540

TCTGACTACA ACTACTTGGC CATGGGTAAC TGCATAACCT GTGAGGGCCG GGTGGACAGC 600

CAGGAGTACG CCAACATCCG CTCCGCCATG AAGGTGCTCA TGTTCACTGA CACCGAGAAC 660

TGGGAGATCT CGAAGCTCCT GGCTGCCATC CTGCACCTGG GCAACCTGCA GTATGAGGCA 720

CGGACATTTG AAAACCTGGA TGCCTGTGAG GTTCTCTTCT CCCCATCGCT GGCCACAGCT 780

GCATCCCTGC TTGAGGTGAA CCCCCCAGAC CTGATGAGCT GCCTGAATAG CCGCACCCTC 840

ATCACCCGCG GGGAGACGGT GTCCACCCCA CTGAGCAGGG TAACAGGACT GGACGTGCGC 900

GACGCCTTCG TAAAGGGGAT CTACGGGCGG CTGTTCGTGT GGATTGTGGA CAAGATCAAC 960

GCAGCAATTT ACAAGCCTCC CTCCCAGGAT GTGAAGAACT CTCGCAGGTC CATCGGCCTC 1020

CTGGACATCT TTGGGTTTGA GAACTTTGCT GTGAACAGCT TTGAGCAGCT CTGCATCAAC 1080

TTCGCCAATG AGCACCTGCA GCAGTTCTTT GTGCGGCACG TGTTCAAGCT GGAGCAGGAG 1140

GAATATGACC TGGAGAGCAT TGACTGGCTG CACATCGAGT TCACTGACAA CCAGGATGCC 1200

CTGGACATGA TTGCCAACAA GCCCATGAAC ATCATCTCCC TCATCGATGA GGAGAGCAAG 1260

TTCCCCAAGG GCACAGACAC CACCATGTTA CACAAGCTGA ACTCCCAGCA CAAGCTCAAC 1320

GCCAACTACA TCCCCCCCAA GAACAACCAT GAGACCCAGT TTGGCATCAA CCATTTTGCA 1380

GGCATCGTCT ACTATGAGAC CCAAG 1405

(2) INFORMATION FOR SEQ ID NO: 2:

(i) SEQUENCE CHARACTERISTICS:
(A) LENGTH: 2092 base pairs
(B) TYPE: nucleic acid
(C) STRANDEDNESS: double
(D) TOPOLOGY: linear

(ii) MOLECULE TYPE: cDNA

(iv) ANTI-SENSE: NO

(xi) SEQUENCE DESCRIPTION: SEQ ID NO: 2:

ACGTATACGG GCTCCATCCT GGTGGCTGTG AACCCCTACC AGCTGCTCTC CATCTACTCG 60

CCAGAGCACA TCCGCCAGTA TACCAACAAG AAGATTGGGG AGATGCCCCC CCACATCTTT 120

GCCATTGCTG ACAACTGCTA CTTCAACATG AAACGCAACA GCCGAGACCA GTGCTGCATC 180

ATCAGTGGGG AATCTGGGGC CGGGAAGACG GAGAGCACAA AGCTGATCCT GCAGTTCCTG 240

GCAGCCATCA GTGGGCAGCA CTCGTGGATT GAGCAGCAGG TCTTGGAGGC CACCCCCATT 300

CTGGAAGCAT TTGGGAATGC CAAGACCATC CGCAATGACA ACTCAAGCCG TTTCGGAAAG 360
TACATCGACA TCCACTTCAA CAAGCGGGGC GCCATCGACG GCGCGAAGAT TGAGCAGTAC 420
CTGCTGGAAA AGTCACGTGT CTGTCGCCAG GCCCTGGATG AAAGGAACTA CCACGTGTTC 480
TACTGCATGC TGGAGGGTAT GAGTGAGGAT CAGAAGAAGA AGCTGGGCTT GGGCCAGGCC 540
TCTGACTACA ACTACTTGGC CATGGGTAAC TGCATAACCT GTGAGGGCCG GGTGGACAGC 600
CAGGAGTACG CCAACATCCG CTCCGCCATG AAGGTGCTCA TGTTCACTGA CACCGAGAAC 660
TGGGAGATCT CGAAGCTCCT GGCTGCCATC CTGCACCTGG GCAACCTGCA GTATGAGGCA 720
CGGACATTTG AAAACCTGGA TGCCTGTGAG GTTCTCTTCT CCCCATCGCT GGCCACAGCT 780
GCATCCCTGC TTGAGGTGAA CCCCCCAGAC CTGATGAGCT GCCTGAATAG CCGCACCCTC 840
ATCACCCGCG GGGAGACGGT GTCCACCCCA CTGAGCAGGG TAACAGGACT GGACGTGCGC 900
GACGCCTTCG TAAAGGGGAT CTACGGGCGG CTGTTCGTGT GGATTGTGGA CAAGATCAAC 960
GCAGCAATTT ACAAGCCTCC CTCCCAGGAT GTGAAGAACT CTCGCAGGTC CATCGGCCTC 1020
CTGGACATCT TTGGGTTTGA GAACTTTGCT GTGAACAGCT TTGAGCAGCT CTGCATCAAC 1080
TTCGCCAATG AGCACCTGCA GCAGTTCTTT GTGCGGCACG TGTTCAAGCT GGAGCAGGAG 1140
GAATATGACC TGGAGAGCAT TGACTGGCTG CACATCGAGT TCACTGACAA CCAGGATGCC 1200
CTGGACATGA TTGCCAACAA GCCCATGAAC ATCATCTCCC TCATCGATGA GGAGAGCAAG 1260
TTCCCCAAGG GCACAGACAC CACCATGTTA CACAAGCTGA ACTCCCAGCA CAAGCTCAAC 1320
GCCAACTACA TCCCCCCCAA GAACAACCAT GAGACCCAGT TTGGCATCAA CCATTTTGCA 1380
GGCATCGTCT ACTATGAGAC CCAAGGCTTC CTGGAGAAGA ACCGAGACAC CCTGCATGGG 1440
GACATTATCC AGCTGGTCCA CTCCTCCAGG AACAAGTTCA TCAAGCAGAT CTTCCAGGCC 1500
GATGTCGCCA TGGGCGCCGA GACCAGGAAG CGCTCGCCCA CACTTAGCAG CCAGTTCAAG 1560
CGGTCACTGG AGCTGCTGAT GCGCACGCTG GGTGCCTGCC AGCCCTTCTT TGTGCGATGN 1620
ATCAAGCCCA ATGAGTTCAA GAAGCCCATG CTGTTCGACC GGTACCTGTG NGTGCGCCAG 1680
TTGTGGTACT CAGGAATGAT GGAGACCATC CGAATCCGNC GANCTGGCTA CCCCATCCGA 1740
TACAGATTCG TAGAGTTTGT GGAGCGGGTG CTGCTGCCAG GTGGGAAGCC GGCCTACAAG 1800
CAGGGCGACC TCCGCGGGAC TTGCCAGCGC ATGGCTGAGG CTGTGCTGGG CACCCACGAT 1860
GACTGGCAGA TAGGCAAAAC CAAGATCTTT CTGAAGGACC ACCATGACAT GCTGCTGGAA 1920
GTGGAGCGGG ACAAAGCCAT CACCGACAGA GTCATCCTCC TTCAGAAAGT CATCCGGGGA 1980
TTCAAAGACA GGTCTAACTT TCTGAAGCTG AAGAACGCTG CCACACTGAT CCAGAGGCAC 2040
TGGCGGGGTC ACAACTGTAG GAAGGAACTA CGGGCTGATG CGTCTGGGCT TC 2092

(2) INFORMATION FOR SEQ ID NO: 3:

(i) SEQUENCE CHARACTERISTICS:
(A) LENGTH: 697 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: myosin VII

(xi) SEQUENCE DESCRIPTION: SEQ ID NO: 3:

Thr Tyr Thr Gly Ser Ile Leu Val Ala Val Asn Pro Tyr Gln Leu Leu
1               5                   10                  15

Ser Ile Tyr Ser Pro Glu His Ile Arg Gln Tyr Thr Asn Lys Lys Ile
            20                  25                  30

Gly Glu Met Pro Pro His Ile Phe Ala Ile Ala Asp Asn Cys Tyr Phe
        35                  40                  45

Asn Met Lys Arg Asn Ser Arg Asp Gln Cys Cys Ile Ile Ser Gly Glu
    50                  55                  60

Ser Gly Ala Gly Lys Thr Glu Ser Thr Lys Leu Ile Leu Gln Phe Leu
65                  70                  75                  80

Ala Ala Ile Ser Gly Gln His Ser Trp Ile Glu Gln Gln Val Leu Glu
                85                  90                  95

Ala Thr Pro Ile Leu Glu Ala Phe Gly Asn Ala Lys Thr Ile Arg Asn
            100                 105                 110

Asp Asn Ser Ser Arg Phe Gly Lys Tyr Ile Asp Ile His Phe Asn Lys
        115                 120                 125

Arg Gly Ala Ile Glu Gly Ala Lys Ile Glu Gln Tyr Leu Leu Glu Lys
    130                 135                 140

Ser Arg Val Cys Arg Gln Ala Leu Asp Glu Arg Asn Tyr His Val Phe
145                 150                 155                 160

Tyr Cys Met Leu Glu Gly Met Ser Glu Asp Gln Lys Lys Lys Leu Gly
                165                 170                 175

Leu Gly Gln Ala Ser Asp Tyr Asn Tyr Leu Ala Met Gly Asn Cys Ile
            180                 185                 190

Thr Cys Glu Gly Arg Val Asp Ser Gln Glu Tyr Ala Asn Ile Arg Ser
        195                 200                 205

Ala Met Lys Val Leu Met Phe Thr Asp Thr Glu Asn Trp Glu Ile Ser
    210                 215                 220

Lys Leu Leu Ala Ala Ile Leu His Leu Gly Asn Leu Gln Tyr Glu Ala
225                 230                 235                 240

Arg Thr Phe Glu Asn Leu Asp Ala Cys Glu Val Leu Phe Ser Pro Sr
                245                 250                 255

Leu Ala Thr Ala Ala Ser Leu Leu Glu Val Asn Pro Pro Asp Leu Met
            260                 265                 270

Ser Cys Leu Asn Ser Arg Thr Leu Ile Thr Arg Gly Glu Thr Val Ser
        275                 280                 285

Thr Pro Leu Ser Arg Val Thr Gly Leu Asp Val Arg Asp Ala Phe Val
    290                 295                 300

Lys Gly Ile Tyr Gly Arg Leu Phe Val Trp Ile Val Asp Lys Ile Asn
305                 310                 315                 320

Ala Ala Ile Tyr Lys Pro Pro Ser Gln Asp Val Lys Asn Ser Arg Arg
                325                 330                 335

Ser Ile Gly Leu Leu Asp Ile Phe Gly Phe Glu Asn Phe Ala Val Asn
            340                 345                 350

Ser Phe Glu Gln Leu Cys Ile Asn Phe Ala Asn Glu His Leu Gln Gln
        355                 360                 365

Phe Phe Val Arg His Val Phe Lys Leu Glu Gln Glu Glu Tyr Asp Leu
    370                 375                 380

Glu Ser Ile Asp Trp Leu His Ile Glu Phe Thr Asp Asn Gln Asp Ala
385                 390                 395                 400

Leu Asp Met Ile Ala Asn Lys Pro Met Asn Ile Ile Ser Leu Ile Asp
                405                 410                 415

Glu Glu Ser Lys Phe Pro Lys Gly Thr Asp Thr Thr Met Leu His Lys
            420                 425                 430

Leu Asn Ser Gln His Lys Leu Asn Ala Asn Tyr Ile Pro Pro Lys Asn
        435                 440                 445

Asn His Glu Thr Gln Phe Gly Ile Asn His Phe Ala Gly Ile Val Tyr
    450                 455                 460

Tyr Glu Thr Gln Gly Phe Leu Glu Lys Asn Arg Asp Thr Leu His Gly
465                 470                 475                 480

Asp Ile Ile Gln Leu Val His Ser Ser Arg Asn Lys Phe Ile Lys Gln
                485                 490                 495

Ile Phe Gln Ala Asp Val Ala Met Gly Ala Glu Thr Arg Lys Arg Ser
            500                 505                 510

Pro Thr Leu Ser Ser Gln Phe Lys Arg Ser Leu Glu Leu Leu Met Arg
        515                 520                 525

Thr Leu Gly Ala Cys Gln Pro Phe Phe Val Arg Xaa Ile Lys Pro Asn
    530                 535                 540

Glu Phe Lys Lys Pro Met Leu Phe Asp Arg Tyr Leu Xaa Val Arg Gln
545                 550                 555                 560

Leu Trp Tyr Ser Gly Met Met Glu Thr Ile Arg Ile Arg Arg Xaa Gly
                565                 570                 575

Tyr Pro Ile Arg Tyr Arg Phe Val Glu Phe Val Glu Arg Val Leu Leu
            580                 585                 590

Pro Gly Gly Lys Pro Ala Tyr Lys Gln Gly Asp Leu Arg Gly Thr Cys
        595                 600                 605

Gln Arg Met Ala Glu Ala Val Leu Gly Thr His Asp Asp Trp Gln Ile
    610                 615                 620

Gly Lys Thr Lys Ile Phe Leu Lys Asp His His Asp Met Leu Leu Glu
625                 630                 635                 640

Val Glu Arg Asp Lys Ala Ile Thr Asp Arg Val Ile Leu Leu Gln Lys
                645                 650                 655

Val Ile Arg Gly Phe Lys Asp Arg Ser Asn Phe Leu Lys Leu Lys Asn

660                 665                 670

Ala Ala Thr Leu Ile Gln Arg His Trp Arg Gly His Asn Cys Arg Lys
        675                 680                 685

Glu Leu Arg Ala Asp Ala Ser Gly Leu
    690                 695

(2) INFORMATION FOR SEQ ID NO: 4:

(i) SEQUENCE CHARACTERISTICS:
(A) LENGTH: 1349 base pairs
(B) TYPE: nucleic acid
(C) STRANDEDNESS: double
(D) TOPOLOGY: linear

(ii) MOLECULE TYPE: cDNA

(iv) ANTI-SENSE: NO

(xi) SEQUENCE DESCRIPTION: SEQ ID NO: 4:

ATGAGGAGGA GAAGAAGAAA CTGGGCCTAG GCCAGGCCGC TGACTACAAC TACTTGGCCA 60

TGGGTAACTG CATCACCTGT GAGGGCCGCG TGGACAGTCA GGAGTATGCC AACATCCGCT 120

CTGCCATGAA GGTTCTCATG TTCACAGACA CGGAGAACTG GGAGATCTCG AAGCTTCTGG 180

CTGCCATCCT ACACATGGGC AATCTGCAGT ATGAGACCCG GACATTTGAG AACTTGGATG 240

CGTGTGAAGT CCTCTTCTCC CCATCGCTGC CACGGCCAGT TCTCTGCTCG AGGTGAACCC 300

CCCAGACCTG ATGAGCTGCC TCACCAGCCG CACCCTCATC ACCCGTGGGG AGACAGTGTC 360

CACCCCTCTC AGCAGGGAGC AGGCACTGGA TGTGCGAGAT GCCTTTGTCA AGGGACTCTA 420

TGGGCGGCTC TTTGTGCTGG ATTGTGGAGA AGATCAACGC AGCAATCTAC AAGCCACCCC 480

CTGGAAGTGA AGAACTCTCG CCGGTCCATC GGTCTCCTGG ACATCTTTGG ATTTGAGAAC 540

TTCACTGTGA ACAGCTTCGA GCAGCTCTGC ATTAACTTTG CCAATGAGCA CCTGCAGCAA 600

TTCTTCGTGC GGTACGTGTT CAAGCTGGAG CAGGAGGAGT ACGACCTGGA GAGCATCGAC 660

TGGTTGCACA TTGAGTTCAC TGACAACCAG GAAGCACTGG ACATGATTGC CAACCGGCCT 720

ATGAACGTCA TCTCCCTCAT CGATGAGGAG AGCAAGTTCC CCAAGGGCAC GGATGCCACC 780

ATGCTGCATA AGCTGAACTC ACAGCACAAG CTCAATGCCA ACTACGTGCC ACCCAAGAAC 840

AGCCACGAGA CCAGTTGGAA TCAACCACTT TGCGGTGTTG TCTATTATGA GAGTCAAGGC 900

TTCCTGGAGA AGAACCGAGA CACCTTGCAT GGGACATCAT CCAGCTGGTC CACTCTTCCC 960

GGAAACAAGT TCGTAAAGCA GATTTTCCAG GCTGATGTTG CCATGGGTGC CGAGACCAGG 1020

AAGCGCTCGC CTACACTCAG CAGCCAGTTC AAGCGGTCTC TGGAGCTGCG TATGCGCACA 1080

CTGGGCGCCT GCCAGCCCTA CTTTGTGCGT TGTATCAAAC CCACGAATGA GTCAAGAAAC 1140

CCATGCTCTT CGGACCGGGC TCTTCTGTGT ACGCCAGCTC GCGATATTCG GGCATGATGG 1200

AGACGGATCC CGATCCGCCA CGCAGGCTAC CCCATCGCTA CAGCTTTGTG GTGTTTGTGG 1260

AGCGCTACCG GGTACTGCTG CCTGGCGTGA AGCCAGCATG CAAGCAGGGT GACCTCCGAG 1320

GGACATGCCA GCGCATGGCT GAGGCTGTG 1349

(2) INFORMATION FOR SEQ ID NO: 5:

(i) SEQUENCE CHARACTERISTICS:
(A) LENGTH: 1255 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 5:

ACGTACACAG GTTCCATCCT GGTGGCCGTG AACCCCTACC AGCTGCTCTC CATCTACTCG 60

CCAGAGCACC ACTCCGCCAG TACACCAACA AGAAGATAGG GGAGATGCCC CCCACATCTT 120

CGCCATTGCT GACAACTGCT ACTTCAACAT GAAACGCAAC AACCGGGACC AGTGCTGTAT 180

TATCAGGTAG GTGGCCCGGC CTTACTCCCA GTGAGAGGCA CAGTTCTGCT GCCTTGCTAA 240

CACACTAGGA ATTGTTCCAT CGTTAGACTG CCAGGGGGCT CCCCGGGAGC TGGATGTGCC 300

TCTGTGCTGT GTCATAGTGT ATGGAGTCAC CCCCACATTC CAGATCTTAC AGTTCTTTTC 360

TCTTAACCCT ACAAAGACTG ATGCTAGCTT TTCAGGTCAA CACTGTCTCC TGAGGGTTCC 420

TATTAAGTGA TGGTCAGTTG AAATCTGTCT TCTTTGCTCT TGGGCAAGGG AAGAGGTCAA 480

AACATCAGTT GAGCAGTTCG CATGATTCAT ATTCTTGACC AGGAGGGATG GGGGTGTGGC 540

AGAGCCTTTC AGCCTGAAGA CTCGACCTAT TGTAACAACT GCGGCTAGTG TCAACAAGCC 600

AAGGCTGAGG ACTTGCCCGT GACTCCCACC CTCTGCAGCG GGGAGTCGGG AGCTGGCAAG 660

ACAGAGAGCA CAAAGTTGAT CCTGCAGTTC CTGGCAGCCT ACAGTGGACA GCACTCATGG 720

ATCGAGCAGC AGGAGCTGGA GGCCACCCCG ATCCTGGAAG GTAAGGCCAG GTTCTGAAGG 780

TGAGAGCCCA GGCTGGCACT GGTCTACCCA GGCTCACCTG TGATCCCTGC CACCCTGCAG 840

CATTTGGGAA CCCCAAGACC ATCCGAACGA CAACTCTAGC CGCTTTGGAA GTACATTGAC 900

ATCCACTTTA ACAAGCGTGG TGCCATCGAG GGCGCCAAAA TAGAGCAATA CCTGCTGGAA 960

GAAGTCACGG TGCTGCCGCC AGTGAGCCCT GCTCCCAGTG ATATCAGAGC CTGGAGCAGC 1020

TGGGTGGGCA CCAGGGTCTG GGCCATCTGT TAGCACACTG TACTTCCATC TGTCCTTGAA 1080

GTGTTCAAGG TCCCCTATGA GACCCCTGCT CATGTGTGTT CCCAATCCTG GTGCCCACTT 1140

CCAGGCCCCT GACGAGAGGA ACTATCAGCT GTTCTACTGT ATGCTGGAGG GCATGAATGA 1200

GGAGGAGAAG AAGAAACTGG GCTAGGCAGG CGTCGACTAC AACGACTTGG CCATG 1255

(2) INFORMATION FOR SEQ ID NO: 6:

(i) SEQUENCE CHARACTERISTICS:
(A) LENGTH: 631 amino acids
(B) TYPE: amino acid
(C) STRANDEDNESS: single

(D) TOPOLOGY: linear

(ii) MOLECULE TYPE: protein

(vi) ORIGINAL SOURCE:
(A) ORGANISM: myosin VII

(xi) SEQUENCE DESCRIPTION: SEQ ID NO: 6:

Thr Tyr Thr Gly Ser Ile Leu Val Ala Val Asn Pro Tyr Gln Leu Leu
1               5                   10                  15

Ser Ile Tyr Ser Pro Glu His Ile Arg Gln Tyr Thr Asn Lys Lys Ile
            20                  25                  30

Gly Glu Met Pro Pro His Ile Phe Ala Ile Ala Asp Asn Cys Tyr Phe
        35                  40                  45

Asn Met Lys Arg Asn Asn Arg Asp Gln Cys Cys Ile Ile Arg Gly Glu
    50                  55                  60

Ser Gly Ala Gly Lys Thr Glu Ser Thr Lys Leu Ile Leu Gln Phe Leu
65                  70                  75                  80

Ala Ala Ile Ser Gly Gln His Ser Trp Ile Glu Gln Gln Val Leu Glu
                85                  90                  95

Ala Thr Pro Ile Leu Glu Ala Phe Gly Asn Ala Lys Thr Ile Arg Asn
            100                 105                 110

Asp Asn Ser Ser Arg Phe Gly Lys Tyr Ile Asp Ile His Phe Asn Lys
        115                 120                 125

Arg Gly Ala Ile Glu Gly Ala Lys Ile Glu Gln Tyr Leu Leu Glu Lys
    130                 135                 140

Ser Arg Val Cys Arg Gln Ala Pro Asp Glu Arg Asn Tyr His Val Phe
145                 150                 155                 160

Tyr Cys Met Leu Glu Gly Met Asn Glu Glu Glu Lys Lys Lys Leu Gly
                165                 170                 175

Leu Gly Gln Ala Ala Asp Tyr Asn Tyr Leu Ala Met Gly Asn Cys Ile
            180                 185                 190

Thr Cys Glu Gly Arg Val Asp Ser Gln Glu Tyr Ala Asn Ile Arg Ser
        195                 200                 205

Ala Met Lys Val Leu Met Phe Thr Asp Thr Glu Asn Trp Glu Ile Ser
    210                 215                 220

Lys Leu Leu Ala Ala Ile Leu His Met Gly Asn Leu Gln Tyr Glu Ala
225                 230                 235                 240

Arg Thr Phe Glu Asn Leu Asp Ala Cys Glu Val Leu Phe Ser Pro Ser
                245                 250                 255

Leu Ala Thr Ala Ala Ser Leu Leu Glu Val Asn Pro Pro Asp Leu Met
            260                 265                 270

Ser Cys Leu Thr Ser Arg Thr Leu Ile Thr Arg Gly Glu Thr Val Ser
        275                 280                 285

Thr Pro Leu Ser Arg Glu Gln Ala Leu Asp Val Arg Asp Ala Phe Val
    290                 295                 300

Lys Gly Ile Tyr Gly Arg Leu Phe Val Trp Ile Val Glu Lys Ile Asn
305                 310                 315                 320

Ala Ala Ile Tyr Lys Pro Pro Pro Leu Glu Val Lys Asn Ser Arg Arg
                325                 330                 335

Ser Ile Gly Leu Leu Asp Ile Phe Gly Phe Glu Asn Phe Thr Val Asn
            340                 345                 350

Ser Phe Glu Gln Leu Cys Ile Asn Phe Ala Asn Glu His Leu Gln Gln
        355                 360                 365

Phe Phe Val Arg His Val Phe Lys Leu Glu Gln Glu Glu Tyr Asp Leu
    370                 375                 380

Glu Ser Ile Asp Trp Leu His Ile Glu Phe Thr Asp Asn Gln Glu Ala
385                 390                 395                 400

Leu Asp Met Ile Ala Asn Arg Pro Met Asn Val Ile Ser Leu Ile Asp
                405                 410                 415

Glu Glu Ser Lys Phe Pro Lys Gly Thr Asp Ala Thr Met Leu His Lys
            420                 425                 430

Leu Asn Ser Gln His Lys Leu Asn Ala Asn Tyr Val Pro Pro Lys Asn
        435                 440                 445

Ser His Glu Thr Gln Phe Gly Ile Asn His Phe Ala Gly Val Val Tyr
    450                 455                 460

Tyr Glu Ser Gln Gly Phe Leu Glu Lys Asn Arg Asp Thr Leu His Gly
465                 470                 475                 480

Asp Ile Ile Gln Leu Val His Ser Ser Arg Asn Lys Phe Val Lys Gln
                485                 490                 495

Ile Phe Gln Ala Asp Val Ala Met Gly Ala Glu Thr Arg Lys Arg Ser
            500                 505                 510

Pro Thr Leu Ser Ser Gln Phe Lys Arg Ser Leu Glu Leu Leu Met Arg
        515                 520                 525

Thr Leu Gly Ala Cys Gln Pro Phe Phe Val Arg Cys Ile Lys Pro Asn
    530                 535                 540

Glu Phe Lys Lys Pro Met Leu Phe Asp Arg His Leu Cys Val Arg Gln
545                 550                 555                 560

Leu Arg Tyr Ser Gly Met Met Glu Thr Ile Arg Ile Arg His Ala Gly
                565                 570                 575

Tyr Pro Ile Arg Tyr Ser Phe Val Glu Phe Val Glu Arg Tyr Arg Val
            580                 585                 590

Leu Leu Pro Gly Val Lys Pro Ala Tyr Lys Gln Gly Asp Leu Arg Gly
        595                 600                 605

Thr Cys Gln Arg Met Ala Glu Ala Val Leu Gly Thr His Asp Asp Trp
    610                 615                 620

Gln Ile Gly Lys Thr Lys Ile
625                 630

## Description

The present invention concerns a nucleotide sequence and the corresponding peptide sequence, the nucleotide sequence being that of the human gene associated with non syndronic deafness, called DFNB2 and of the human gene associated with USHER1B syndrome called USH1B, as well as their diagnostic and therapeutic applications.

The invention also relates to the homologous mouse sequence which is part of the shaker-1 gene (sh1) involved in hearing impairment and relates also to the nucleotide sequences which hybridize under stingent conditions to the human gene DFNB2/USHER 1B or to the murine gene shaker 1. The invention also relates to the genetic sequences corresponding to the cDNAs contained in the genomic DNA, said genomic DNA possibly including the regulatory regions of the gene.

According to the instant invention, genes involved in hearing impairment have been for the first time identified.

The present invention represents also the first evidence that genes involved in hearing impairment encode unconventional myosin molecules involved in auditory transduction.

Usher syndrome (USH) represents the association of a hearing impairment with retinitis pigmentosa (1) and is the most frequent cause of deaf-blindness in humans. USH is inherited as an autosomal recessive trait. Histopathological studies have revealed, in some patients, an abnormal organisation of the microtubules in the anoxeme of the photoreceptors cells (connecting cilium) (4-6), the nasal ciliar cells (7) and the sperm cells (5), as well as a widespread degeneration of the organ of Corti (8). Three distinct clinical subtypes have been described. USH type 1 (USH1) is characterized by a profound congenital sensorineural hearing loss, constant vestibular dysfunction and prepubertal onset of retinitis pigmentosa. Three different genes responsible for USH1 have been mapped (9-11). The USH1B gene maps to 11q13.5 (10) and accounts for about 75 % of USH1 patients (2, 3).

The mouse deafness shaker-1 (sh1) mutation has been localised in the homologous murine region (12, 13).

The inventors of the present invention have identified the gene candidate for shaker-1 and demonstrated that it encodes an unconventional myosin. Taking into account the cytoskeletal anomalies described in USH patients, the identification of sh1 led them to consider the human homologue thereof as a good candidate for USH1B.

Furthermore, according to the work of the inventors, a gene called DFNB2 (previously called NSRD2 (14), which maps to the same chromosomal region as USH1B appears to be the human equivalent of the sh1 gene and is probably an allele of USH1B.

According to a first aspect, the instant invention provides a DNA comprising all or part of a nucleotide sequence selected from SEQ ID N° 1, sequences hybridizing under stingent conditions to all or part of SEQ ID N° 1 and any other sequence which code for the same amino acid sequence as encoded by the nucleotide sequence sequence SEQ ID N° 1 due to the degeneracy of the genetic code.

The conditions of stringency are as defined by SAMBROOK et al, (30).
For exemple, prefered conditions of stringency consist in a "Church" buffer at 65°C, the buffer containing a phosphate buffer 0,5M pH 7,2 SDS 7%, EDTA 1mM.

The DNA as defined therin may consist of cDNA of sequence SEQ ID N° 1 or of cDNA of sequence SEQ ID N° 2 of human origin. The DNA may also consist of cDNA of sequence SEQ ID N°4 or of genomic DNA of sequence SEQ ID N° 5 of murine origin.

According to another aspect, the present invention also provides a vector which may consist of a cloning vector or an expression vector containing a DNA sequence as defined previously.

Preferred cloning vectors are the following plasmids or the following phages containing fragments of DNA as defined previously, deposited at the C.N.C.M. (Collection Nationale de Cultures de Microorganismes), on February 3, 1995, under the BUDAPEST Treaty.
- I-1527 containing a myosin VII A cDNA fragment starting from nucleotide 1495 and extending on about 3kb .
- I-1528 containing a myosin VII A cDNA fragment starting from nucleotide 1293 and extending on about 800 base pairs.
- I-1526 containing a human genomic DNA of about 3,5kb, containing 5 exons (1 to 5) of the myosin VII A gene.
- I-1525 containing a human genomic DNA containing 5 exons (6 to 10) of the myosin VII A gene.
- I-1529 obtained from a λZap phage, containing a murine genomic DNA fragment encompassing part of myosin VII A gene.
-I-1530 obtained from a λZap phage, containing a murine genomic DNA fragment encompassing part of myosin VII A gene.

The preferred sequences are the genomic sequences which contain the genetic sequences corresponding to the cDNA and regulatory sequences or the non coding sequences (introns).

The instant invention also pertains to any DNA insert originating from USH1B/DFNB2 contained in the above-mentioned plasmids.

According to a further aspect, the present invention also provides a nucleic probe specific for the human USH1B or DFNB2 genes or the murine sh1 gene, comprising at least 20 consecutive nucleotides selected from the nucleotide sequences as defined above.

The subject matter of the present invention also consists in oligonucleotide primers for the specific amplification of the human USH1B/DFNB2 genes or the murine sh1 gene or of fragments thereof, comprising fragments of nucleotide sequences as defined above.

The primers preferably have a length of 18 to 30 nucleotides and, preferably, of 18 to 22 nucleotides.

One of the two primers is complementary to the (+) strand of the template and the other primer is complementary to the (-) strand. It is important that these primers do not possess a secondary structure or a mutually complementary sequence. Furthermore, the length and the sequence of each primer should be selected in such a manner that the primers do not hybridize with other nucleic acids present in the biological sample.

The amplimers selected as specific primers for the amplification of nucleotide sequences can be selected for example by following the method described by Griffai et al. (40).

Advantageously, the primers can also consist in oligonucleotides of 18 to 30 nucleotides, preferably 21 to 25, selected from the intervening sequences flanking the exons.

The amplified fragments can be identified after an agarose or polyacrylamide gel electrophoresis or after a capilliary electrophoresis or alternatively after a chromatographic technique (gel filtration, hydrophobic or ion-exchange chromatography). The specificity of the amplification can be controlled by molecular hybridization using as probes the sequences as defined above or plasmids containing such sequences. These probes may be labeled by radioactive elements or otherwise by non-radioactive molecules.

The subject-matter of the present invention is also a nucleotide probe specific for the human USH1B/DFNB2 genes or the murine sh1 gene comprising at least 20 consecutive nucleotides selected from the nucleotide sequences as defined above.

The non-labelled sequences can be used directly as probes, however, the nucleic acid sequences are generally labelled with a radioactive element (³²P, ³⁵S, ³H, ¹²⁵I) or with a non-radioactive molecule (biotin, acetylaminofluorene, digoxigenin, 5-bromodeoxyuridin) in order to obtain probes which can be used for numerous applications. In this latter case, it will be possible to use one of the labelling methods described in FR 2,422,956 and FR 2,518,755.

The hybridization technique can be performed in various ways (Matthews et al. (19)). The most general method consists in immobilizing the DNA from the biological sample on a support (nitrocellulose, nylon, polystyrene and the like) and in incubating, under well defined conditions, the immobilized target DNA with the probe DNA. After hybridization, the excess probe is removed and the hybrid molecules formed are detected by the appropriate method (measurement of the radioactivity, of the fluorescence or of the enzymatic activity linked to the probe and the like).

When a sufficient quantity of DNA can be extracted from samples to be analysed, the sequences described above can be used to detect and identify the gene or a fragment of gene associated with syndromic or non syndromic deafness directly in these samples. In the opposite case, a rapid culture in liquid medium can be performed before extraction of the DNA, or, alternatively, the small quantity of DNA extracted from the sample can be subjected to the PCR technique.

According to a further aspect, the present invention also provides a polypeptide or protein encoded by a nucleotide sequence as defined above, namely the peptide sequence of the human or murine non-conventional myosin VII sequence corresponding respectively to SEQ ID N° 1 and SEQ ID N° 4 as well as a peptide sequence derived from that sequences wherein the functionality of the naturally occuring protein is substantially maintained.

The invention also includes the purified protein obtainable by the expression of the cDNA or genomic DNA in an expression vector by using technics which are known to the skilled man in the artsuch as those described in WO/91 09955.

Sequences of that type may differ from the sequence of the naturally occuring protein by insertion, deletion or substitution of one or more amino acids.

The subject matter of the present invention is further a monoclonal or a polyclonal antibody recognizing a peptide sequence as defined above.

The subject matter of the present invention is also a method of detection of a genetic abnormality linked to syndromic or non syndromic deafness in a biological sample containing human DNA comprising the following steps :
a) bringing the biological sample containing the DNA into contact with a pair of specific primers as defined above, the human DNA contained in the sample having been optionally rendered accessible to hybridization and under conditions permitting a hybridization of the primers with the human DNA contained in the biological sample;
b) amplification of the human DNA;
c) revealing the amplification products by appropriate techniques;
d) optionally detecting a mutation or a deletion by appropriate techniques.

The mutations can be detected by direct sequencing or single-strand conformation polymorphism (SSCP) analysis as described by M. Orita et al. (23), or by that of denaturing gradient gel electrophoresis (DGGE) such as that described by R. Myers et al. (29), these procedures being performed on the amplification products or digestion fragments thereof.

The deletions can be detected by determination of the length of the amplified fragments, for example by a polyacrylamide gel electrophoresis or alternatively by determination of the sequence of the amplified fragment.

The method of detection of a genetic abnormality linked to syndromic or non syndromic deafness can be performed for example on a sample containing cells of human foetus, after culturing the said cells.

The method as defined herein is particularly appropriated for the diagnosis of USHER1B syndrom or of non syndromic deafness associated with DFNB2.

Since the present invention provides envidence that genetic deafness is associated with an alteration of a protein consisting in a non conventional myosin, it is highly possible that other types of genetic syndromic or non syndromic deafnesses are associated with a non functional gene coding for a non-conventional myosin.

By non-conventional myosin in the sense of the instant invention there is to be understood a myosin previously called from type I, (i.e. myosins which do not belong to the class of skelettal muscular myosins).

Thus, according to a further aspect, the invention provides a method of in vitro diagnosis of a human syndromic or non-syndromic deafness consisting in determining a mutation or a deletion of one or more base(s) of the human gene coding for an unconventional myosin molecule.

The subject of the present invention is also a kit for the detection of a genetic abnormality linked to genetic syndromic or non syndromic deafness in a biological sample, comprising the following elements :
- a pair of specific primers as defined above,
- the reagents necessary for carrying out a DNA amplification,
- a component which makes it possible to detect a mutation or a deletion of the genomic DNA.

According to a further aspect, the present invention also provides a pharmaceutical composition comprising all or part of the nucleotide sequence of a gene encoding an unconventional myosin molecule, particularly a DNA sequence as defined above.

The pharmaceutical composition consists preferably in a naked DNA sequence or a total or part of genomic DNA or a cDNA or a vector containing a DNA sequence as mentioned above for transferring into somatic or germ cells of a patient suffering from syndromic or non syndromic deafness associated to an alteration of said DNA the functional DNA or a functional fragment thereof contained in the vector.

For example, in the case of DFNB2 or USHER1B syndrom the vector may be an appropriate vector for transferring DNA of interest in an ear and/or eye cell where the gene of interest is normally expressed, the DNA being preferably, but not necessarily, under the control of the promoter of USH1B gene or DFNB2 gene.

The instant invention also concerns a method of gene therapy consisting in transferring in somatic or germ cells of a patient suffering from genetic syndromic or non syndromic deafness a DNA or fragments of a DNA able to restaure the normal function of the gene responsible for the deafness.

The instant invention also encompasses a transgenic animal consisting in non human mammalian animal whose germ cells and somatic cells contain a DNA as defined above as a result of chromosomal incorporation into the animal genome, or into an acestor of said animal, of said DNA.

The invention also encompasses cell lines transfected with genomic sequences or with the corresponding cDNA as previously defined coding for the DFNB2 or USHER1B protein or a fragment thereof ( WO 91/06667, WO 90/11354).

The identification of the USH1B gene (or DFNB2 gene) and the murine homologous gene have been performed according to the following procedures, for the comprehension of which it will be referred to the enclosed Figures wherein :
- Fig. 1 represents the procedure for the gene identification in the sh1 region of mouse chromosome 7;
- Fig. 2 represents the amino acid sequence of head region of mouse myosin VII gene,
- Fig. 3 represents the results of Nothern analysis of the expression product of the mouse myosin VII gene;
- Fig. 4 represents the RT-PCR analysis of myosin VII A expression in human and mouse;
- Fig. 5 represents the nucleotide and deduced amino acid sequences of the two myosin VII A exons of the human gene where mutations associated with USHER1B syndrom were detected;
- Fig. 6 represents the amino acid alignments in different myosins around a 6 bp found in two families.

### I - Identification of the sh1 gene and the corresponding myosin VII protein

The original sh1 mouse mutant carries a spontaneous, recessive mutation causing hyperactivity, head-lossing and circling together with a rapidly-progressive hearing impairment (31, 34). The inner ear shows typical neuroepithelial-type defects (35), involving dysfunction and progressive degeneration of the inner ear sensory epithelia. Even in young mutant mice which show some evidence of cochlear function, responses to sound are never normal, with most mutants showing no eight nerve compound action potentials. These observations indicate that the sh1 gene is expressed in sensory hair cells or cochlear neurons. Vestibular dysfunction accounts for the behaviour of the mutants. Six further sh1 alleles have been recovered, one a spontaneous mutation and five induced by ENU mutagenesis (see Table 1). These new alleles also cause severe vestibular and cochlear dysfunction.

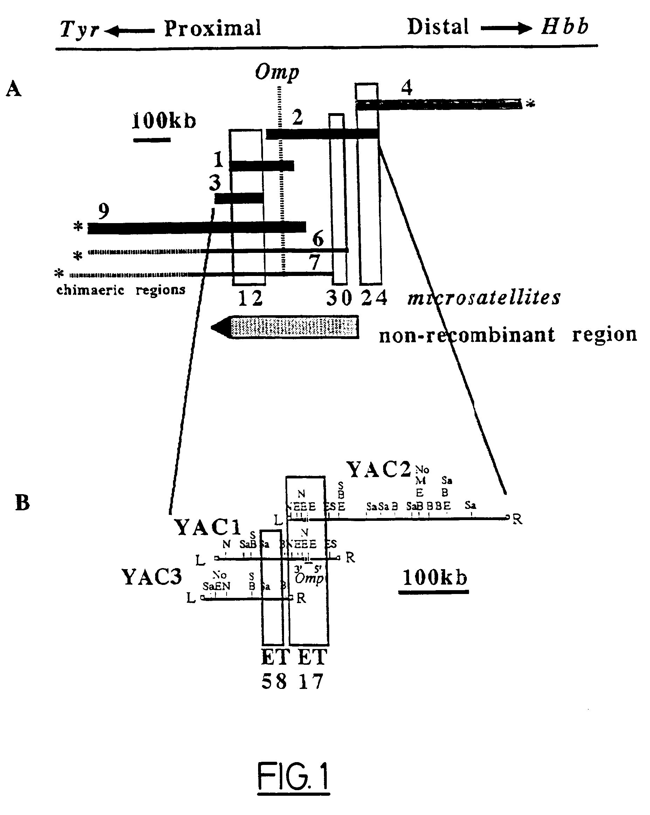

The Olfactory marker protein gene (Omp) has previously been reported as being very tightly linked to the mouse sh1 mutation on mouse chromosome 7. In a backcross of 1,066 progeny segregating for the sh1 mutation, analysis of the flanking markers Tyrosinase (Tyr) and β-globin (IIbb) in the total set of backcross progeny identified a panel of 67 mice that were recombinant in the vicinity of sh1. Analysis of the Omp gene through the recombinant panel demonstrated that Omp is non-recombinant with sh1 and indicated that the sh1 locus is likely to lie within 0.1cM (approximately two hundred kilobases) of the Omp gene. Omp was used a start-point for the construction of a YAC contig across the sh1 region as represented in Fig. 1.

A YAC contig of seven clones was established over a 1.4Mb region surrounding the Omp gene by analysis of three mouse YAC libraries that were available for PCR based screening (the Princeton (41), St. Mary's (42), and MIT (43) mouse YAC libraries). Internal microsatellites were isolated from the first YACs identified - YACs 1 and 2 - by gel purification of YAC DNA via pulsed-field gel electrophoresis (37), digestion with Sau3A and cloning into pBluescript followed by colony screening with a CA repeat. Following sequencing, primers were designed for microsatellites 12, 24 and 30;
12 : TGGTACGGATCTGACGAGACAA/GGTGAGTGCAGCCTGTTCTAGC
24: ACTGTGCCAGCTTCTTTCTCAAAT/TTGGGTGTTTCTCTGTCATGTTCC
30 : GGAAGCCTTTTATGAATTATTACC/CTAAGCTGATTGAGTGTGAAATCAT.
Microsatellites 12, 24 and 30 were used to confirm the internal integrity of the contig in the region of YAC1 anbd YAC2 as well as extend the contig by the isolation of YACs 4, 6, 7 and 9 by PCR screening of the available mouse YAC libraries. Simple sequence length polymorphisms (SSLPs) for microsatellites 12, 24 and 30 were identified beteween the parental strains of the sh1 backcross - CBA/Cash1/sh1 and CB57BI/10 (Ref. 36). SSLP segregation analysis of microsatellites 12, 24 and 30 through the sh1 backcross recombinant panel was used to confirm their location on chromosome 7 and to locate flanking breakpoints to the sh1 gene. Furthermore, unique end clones (identified by asterisks on Fig. 1) were also isolated from the extending YACs 4, 6, 7 and 9 by the PICL/pLUS vector system (44). Subsequently, restriction fragment length variants for these end clones were identified in either the parental strains for the sh1 backcross or, alternatively, a second interspecific backcross segregating markers on mouse chromosome 7 (45). Segregation analysis through either of these crosses was used to assess the location of end clones to chromosome 7. While clones 4 and 9 were non-chimaeric, clones 6 and 7 were chimaeric at their proximal ends. In addition, it has been demonstrated that the distal end clone of YAC 4 was also recombinant in the same backcross mouse as microsatellite 24 confirming that the distal limits to the non-recombinant region had been identified (Fig. 1A).

YAC restriction maps in the vicinity of Omp and the identification and mapping of new genes in this region. [B = BsshII; E = Eag I; M = Mlu I; S = Sst II; Sa = Sal I; No = Not I; [Note a presumed polymorphism between YAC1 and YAC3 for a Sal I site - YAC 1 and YAC 3 were obtained from the Princeton (41) and St. Mary's (42) YAC libraries respectively which were derived from different mouse strains]. Exon trapping of YAC1 was carried out as described previously (37) and identified - two conserved exons RT17 and RT68 that were shown by hybridization analysis to map to the indicated region of YAC1 close to Omp and to the expected overlapping regions of YAC2 and YAC3 respectively.

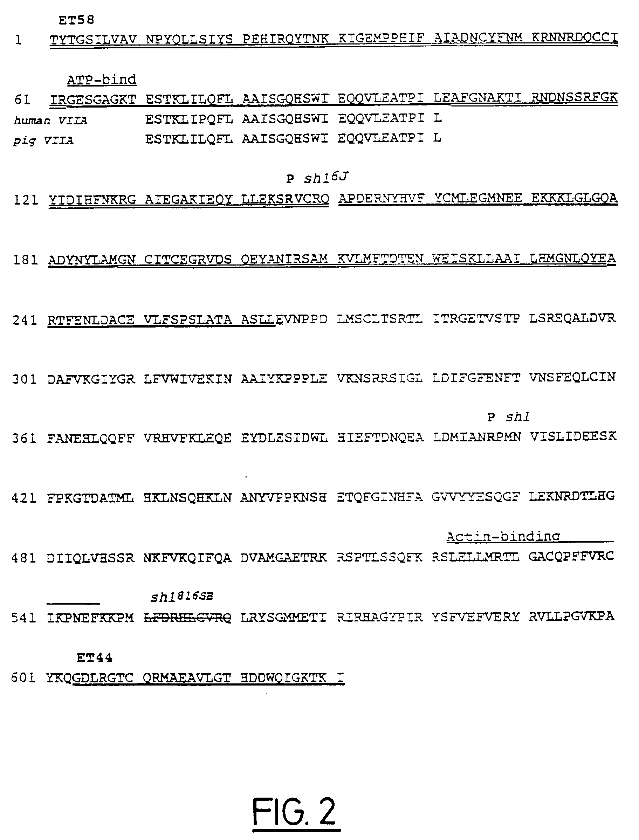

Nine unique exon trap products were recovered from the 175kb YAC1 of which two - ET17 and ET58 - identified conserved sequences and mapped to different regions of the YAC (Ref. 37). ET58 was used to isolate a 4.6 kb clone from a mouse inner ear cDNA library as represented on Fig. 2.

The method used was the following : Myosin head sequence was obtained by sequencing a 4.6kb cDNA homologous to ET58 that was derived by screening a mouse inner ear cDNA library. This unidirectional cDNA library was constructed using the λZAP XR vector (Stratagene). The primary library contained 1.05 x 10⁶ phages and the amplified library had a titer of 9.1 x 10⁹ pfu/ml with 97.7 % being recombinants with an average insert size of 1.1 kb. The 4.6 kb cDNA was unspliced and following sequencing allowed the identification of five complete exons and part of a sixth exon in the N-terminal region of the head. Further head sequence was recovered by RT-PCR using primers developed to exon trap product ET44. An RT-PCR product of approximately 2kb spanning ET58 to ET44 was recovered from kidney RNA and cloned into the PCRII cloning vector (Invitrogen) followed by sequencing . RT-PCR employed Pfu polymerase to ensure maximum fidelity. Further sets of primers were designed from the available head sequence for RT-PCR of kidney RNA from the sh1 mutants. RT-PCR products were cloned into PCRII and multiple clones sequenced for each RT-PCR product. In the case of sh1^{6J}, the mutation was confirmed by identification of a restriction site change associated with the mutation followed by restriction digestion analysis of RT-PCR products of sh1^{6J} and the coisogenic C57BI/KsJ-dbm background strain. An *Afl* III site is destroyed in SH1^{6J}.

Sequence analysis of the 4.6kb cDNA indicated that it encoded a myosin-like protein. However, this cDNA was unspliced and contained only 795bp of coding sequence from the myosin head region comprising 5 complete exons, starting with ET58 at the 5' end, and a portion of a sixth exon troncated by the cloning site at the extreme 3' end. Nevertheless, the available myosin sequence indicated that the ET58 gene encoded a myosin type VII - an unconventional family of myosin molecules (38), whose tail sequence and structure are unknown but for which porcine, human and frog sequences from portions of the head region are available (39). Comparison of protein sequence just C-terminal of the ATP-binding domain with the characteristic motifs that define the other myosin classes (39) in this relatively diverged region demonstrated 100 % identity with pig myosin VIIA and 97 % identity with human myosin VIIA classes as represented on Fig. 2. Subsequent careful comparison of the remaining exon traps from YAC1 with myosin sequences in the database indicated that two further exons, ET29 and ET44, were derived from myosin-like sequences and appeared to be part of the same myosin VII gene derived from the C-terminal region of the myosin head. RT-PCR between ET44 and ET58 produced the expected product of around 2kb and allowed the recovering of the bulk of the head region sequence encompassing 1893bp as represented on Fig. 2.

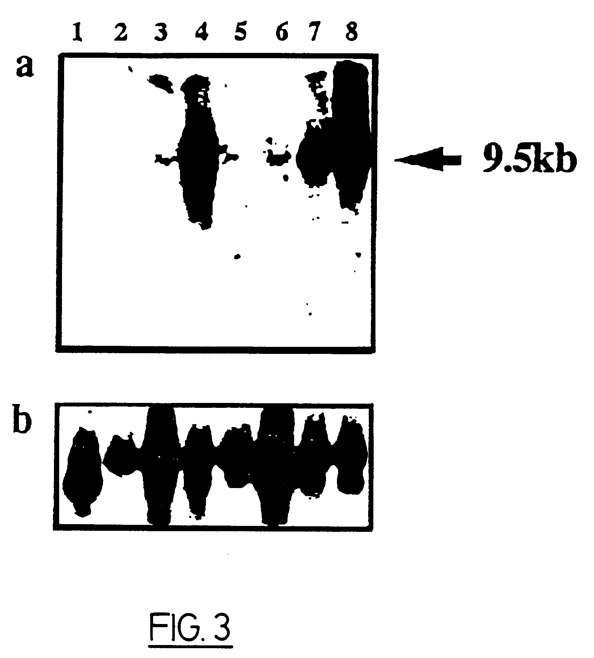

The positions of detected mutations in sh1 alleles are indicated (see Table 1) - sh1 arginine to proline change at position 407 (indicated in bold), sh1^{6J} arginine to proline change at position 145 (indicated in bold) and sh1^{816SB} at positions 551-560 (the 10 amino acid deletion is struck through). In addition, the exon structure where known is indicated (adjacent exons are indicated by alternative double and thick underlining). Five complete exons have been characterized along with part of a sixth exon to position 265 (dotted underlined). The ATP-binding and action-binding sites of the myosin head are also shown as are the characteristic sequence motifs for human and pig myosin VII (Ref. 39) which are aligned with the mouse myosin sequence. Northern analysis detected a myosin VII transcript of approximately 9.5kb in lung (line 4), kidney (line 7) and testis (line 8) as represented on Fig. 3. A very low level of expression was found in skeletal muscle (line 6), heart (line 1), brain (line 2), spleen (line 3) and liver (line 5). In addition, PCR of the ET58 exon from a rat outer hair cell cDNA library produced the expected size product (data not shown).

All seven alleles at the sh1 locus were screened for mutations in the myosin VII gene. Three confirmed mutations are detailed in Table 1. RT-PCR products covering the available coding sequence have been recovered for all seven mutations and sequenced along with the appropriate background strains. The sh1 mutation shows a non conservative arginine to proline change. The sh1^{6J} mutation when compared to the coisogenic background strain C57BL/KsJ-dbm strain shows a non-conservative Arginine to Proline change close to the ATP-binding site of the myosin head. The sh1^{6J} mutation was confirmed by restriction analysis as mentioned for Fig. 2. The altered Arginine residue is highly conserved throughout evolution being found in all myosin genes so far studied. Interestingly, an Arginine to Cysteine mutation at this position in *C. elegans* skeletal muscle myosin shows a slow moving phenotype and some thick filament disroganisation. In the ENU induced mutation sh1^{816SB}, abnormal RT-PCR products were observed using primers in the region of the actin-binding site suggesting the possibility of a splice site mutation. Instead of a normal 215bp PCR product, a smaller PCR product of 185bp and two larger products of 279bp and 318bp were observed. The 185bp PCR product resulted from an in-frame deletion of 10 amino acids just 4 amino acids downstream of the actin-binding site (Fig. 2). The larger PCR products, as expected, contained intron sequence upstream of the deleted 10 amino acids and allowed the identification of a 3' splice site mutation (Table 1) that presumably leads to exon skipping and the observed in-frame 30bp deletion. The larger PCR products presumably arise through upstream cryptic splice sites in the intron and sequencing of the two larger products demonstrated that both would result in premature termination.

There is strong evidence that a myosin motor plays a pivotal role in the phenomenon of hair cell adaptation. This myosin motor is thought to be responsible for adjusting the tip-link tension between stereocilia that controls the gating mechanism of mechanotransduction in the hair cell. A protein of 120 kDa which can be photoaffinity labelled with UPT - a characteristic of myosins - has been found in stereocilia (40). The 120 kDa protein cross-reacts with a bovine adrenal type I myosin antibody. This antibody also labels the tips of stereocilia suggesting that a type I myosin may be a candidate for the adaptation motor. In support of this, a myosin type 1β molecule is found in the bullfrog saccolar macula. Nevertheless, a number of other myosins, including a myosin type VII, have been identified in the bullforg saccular macula. Given the direct involvement in deafness and hearing impairment demonstrated here for myosin VII, it is proposed that this molecule is also an excellent candidate for the adaptation motor.

### II - Identification of the USH1B/DFNB2 gene and the corresponding myosin VII protein

Southern blot hybridisation of a 2 kb fragment from the mouse 4.6kb cDNA comprising five exons of a myosin gene as described above to YAC 965, previously shown to encompass both the USH1B locus and the OMP gene (14), detected a unique 4.3kb EcoRI fragment. This fragment was subsequently isolated from a lambda subclone of this YAC (L3) and sequenced. Sequence analysis revealed the presence of five exons highly homologous to the corresponding mouse exons. Additional exons on the 3' side were found in a 6kb EcoRI fragment of L3 (immediately adjacent to the 4.2kb EcoRI fragment), upon hybridisation of L3 with a 1.1kb mouse cDNA fragment extending 3' from the aforementioned cDNA sequence as described above. These five additional exons were characterised by sequencing M13 subclones of L3.

The human cDNA reconstituted from these ten exons is 1402 bp long and encodes most of the motor head of the putative myosin. It has an overall identity of 90 % with the mouse myosin VIIcDNA. Human and mouse deduced protein sequences are 94 % identical as represented on Fig. 2. Such a strong interspecies conservation has already been reported for several unconventional myosins (15). The existence of two human myosin VII genes has recently been postulated from the identification of two transcription products encoding very similar 31 amino acid polypeptides. The deduced amino acid sequence reported here comprises one of these, referred to as myosin VIIA (16).

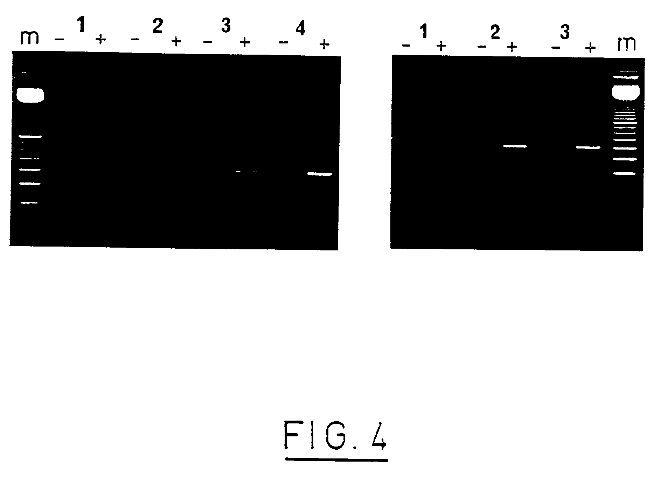

Expression of the myosin VIIA gene was studied in mouse and human by the reverse transcription-polymerase chain reaction (RT-PCR) technique. The results are represented on Fig. 4a (human) and Fig. 4b (mouse).

Total RNA was transcribed using primer 1 (overlapping the junction between the 4th and 5th exons) and then amplified for 40 cycles with primer 1 and primer 2 (ovelapping the 1st and 2nd exons). The primers sequences were the following :
human primer 1 : 5'-TATGCAGTTACCCATGGCCAAG-3';
human primer 2 : 5'-CTGCATCATCAGTGGGGAATC-3';
mouse primer 1 : 5'-CAGGTTGATGCAGTTACCCATG-3';
mouse primer 2 : 5'-CTGTATTATCAGCGGGGAAG-3'.

A 402 bp specific product was detected in human liver (2), kidney (3) and retina (4) but not in brain (1). A 407 bp product is detected in retina (1), cochlea (2) and kidney (3) of mouse, (-)/(+) represent witout/with reverse transriptase. Lane m represent a 100-base ladder (BRL).

RT-PCR products of the expected sizes were detected in the human kidney, liver and retina. In contrast no RT-PCR product was observed in the brain and EBV-transformed lymphocytes (Fig. 4a). In the mouse, expression was detected in the retina, cochlea, kidney and liver (Fig. 4b). The sequences of the PCR products were shown to match those of the isolated cDNA.

### III - Example of mutations in USH1B gene

A search for mutations was undertaken in nine unrelated families in which segregation analysis suggested the involvement of the USH1B gene (3). The five most 5' exons were amplified using intronic flanking primers and the PCT products were sequenced.

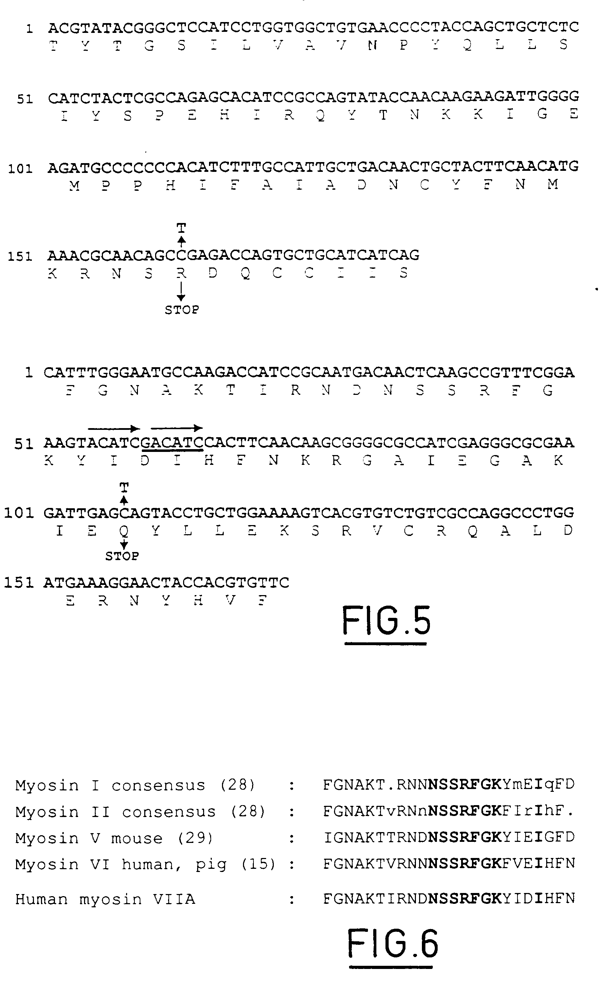

The primers used for sequencing were :
1a : 5'-ACTAACTCCGAGCCAGACC-3';
1b : 5'-GTTACACAGCACAGAGTACATAG-3';
for exon 1 (Fig. 5a)
3a : 5'-GATTGAGCAGCAGGTCTT-3';
3b : 5'-CAATACGGGCAGCAATAC-3'
for exon 3 (Fig. 5b).

The mutations were indicated above and underneath the normal sequences and the 6 bp deletion which was found in two different families is underlined. The arrows show the 5 bp direct repeat. A silent polymorphism was detected in exon 1 at the third position (G->A) and in exon 3 at position 83 (C->T). In three families, mutations were detected in four exons then confirmed on independent PCR products subcloned in M13 vectors. In family 1, a heterozygous C->T mutation in the first exon (nucleotide 173), resulting in a premature stop codon was found in the two affected probands and their mother but not in their father (Fig. 5a). In family 2, a heterozygous C->T transition in the third exon (nucleotide 107), also resulting in a premature stop codon was detected in the affected proband and his mother but not in his unaffected brother and father (Fig. 5b). In families 1 and 3, the same in-frame 6 bp deletion (GACATC) was observed in exon 3 (position 60 to 65), leading to the loss of residues D and I. In family 1, the two affected brothers and their father are heterozygous for the deletion. In family 3, the two affected prebands and their mother but not their father, carry the deleted allele (Fig. 5b). The finding of the same mutation in families originating from different geographical regions suggests that two independent mutational events may have occured, indicating a propensity of this site to mutate. Because this hexanucleotide deletion has occurred un a 11 bp sequence containing two 5 bp direct repeats it is tempting to speculate that either replication slippage or slipped strand mispairing is at the origin of the mutational event. The cosegregation of each of these mutations with the disease and the identification of the mutation in both the alleles of the affected children from family 1, provide strong genetic evidence that the myosin VIIA gene is responsible for USH1B. Moreover, the nonsense mutations found in exons 1 and 3 would result in proteins truncated before the ATP-binding site and the actin-binding site respectively, which most certainly would not be functional. The two amino acid deletion includes an isoleucine which represents a highly conserved residue in conventional (myosin II) as well as unconventional myosins from protozoa to metazoa as represented on Fig. 6 (the heptapeptide containing the mutated arginic and the detected isoleucine are indicated in bold). The recent determination of the X-ray structure of the myosin II, has shown that this residue is located inside a large β sheet (17). Thus this deletion is likely to result in an incorrect folding of the motor domain and consequently in an inactive protein.

The present study is reported in points I and II. It establishes that shaker-1 and USH1B involve the orthologous myosin VIIA genes. However the phenotypes of the original sh1 mutant and the five other sh1 mutants, seem to be different from that of USH1 patients. So far, only cochlear and vestibular defects have been reported in these homozygous mouse mutants, without associated signs of retinal degeneration. It cannot be excluded that the photoreceptor dysfunction, which manifests around prepubertal age in USH1, develops late in mouse life and may not have been recognized for that reason. Alternatively, either another myosin could compensate for a defective myosin VIIA in the mouse retina or the nature of the mutation itself might account for the phenotypic difference. The possibility that the function of myosin VII is partly preserved in sh1 mutants provides an argument for the last proposal. In addition, one the human neurosensorial recessive deafness, not accompanied by retinal dystrophy, DFNB2, which has been mapped to the same chromosomal region as USH1B, may represent the human equivalent of the sh1 mutants. It is noteworthy that although the myosin VIIA gene is expressed in several tissues, its defect appears, both in human and mouse as phenotypically cell-restricted as already observed for mutations in myosin V (20) and myosin VI (21).

Unconventional myosins are motor molecules, with structurally conserved heads which move along actin filaments, using their actin-activated ATPase activity. Their highly divergent tails are expected to be tethered to different macromolecular structures which would move relative to the actin filaments. Since unconventional myosins have been shown to bind to the actin filaments and membranes of microvilli of enterocytes (22), a similar binding of myosin VIIA might exist in the microvilli from inner ear sensory hair cells and/or supporting cells. Myosin VIIA might support either the structural integrity of the microvilli themselves or some specific mechanical aspects of the sensory transduction taking place at the tip of hair cell microvilli (stereocilia); these include the variation of the tip link tension controlling the gating of the cation channels during the adaptation process (23). In that respect, it is of interest that unconventional myosins have been detected at the tip of the stereocilium (24). The primary defect of retinitis pigmentosa has been reported to reside within either the photoreceptor (25) or the pigmentary epithelium cells (26). Therefore, myosin VIIA may also be a component of the microvilli of pigment cells in the retina, where its absence might impair the phagocytosis by these cells of the continually exfoliated membranous discs from rod cells.

Histopathological studies in USH patients have revealed an abnormal microtubular structure of axonemes, especially in photoreceptor cells (4-6). Assuming that some of the patients belong to the USH1B subtype, a direct role of myosin VIIA in the architecture of the photoreceptor connecting cilium must be considered. Interestingly, some actin filaments are located at the distal portion of the cilium (27); an associated myosin might ensure the vectorial transport of membrane components from the inner to the outer segment and might also contribute to the formation of normal microtubular structures. Since cochlear and vestibular hair cells also possess a respectively transient and permanent kinocilium myosin VIIA might play a role in this structure which has been proposed to provide polarity to the hair bundle during development (28).

There are at least seven genes responsible for Usher syndrome, corresponding to four USH1 loci, two USH2 loci and one USH3 locus (2). The present results suggest that abnormalities in othe unconventional myosins or their various binding components might be at the origin of other genetic forms or this dual sensory defect.

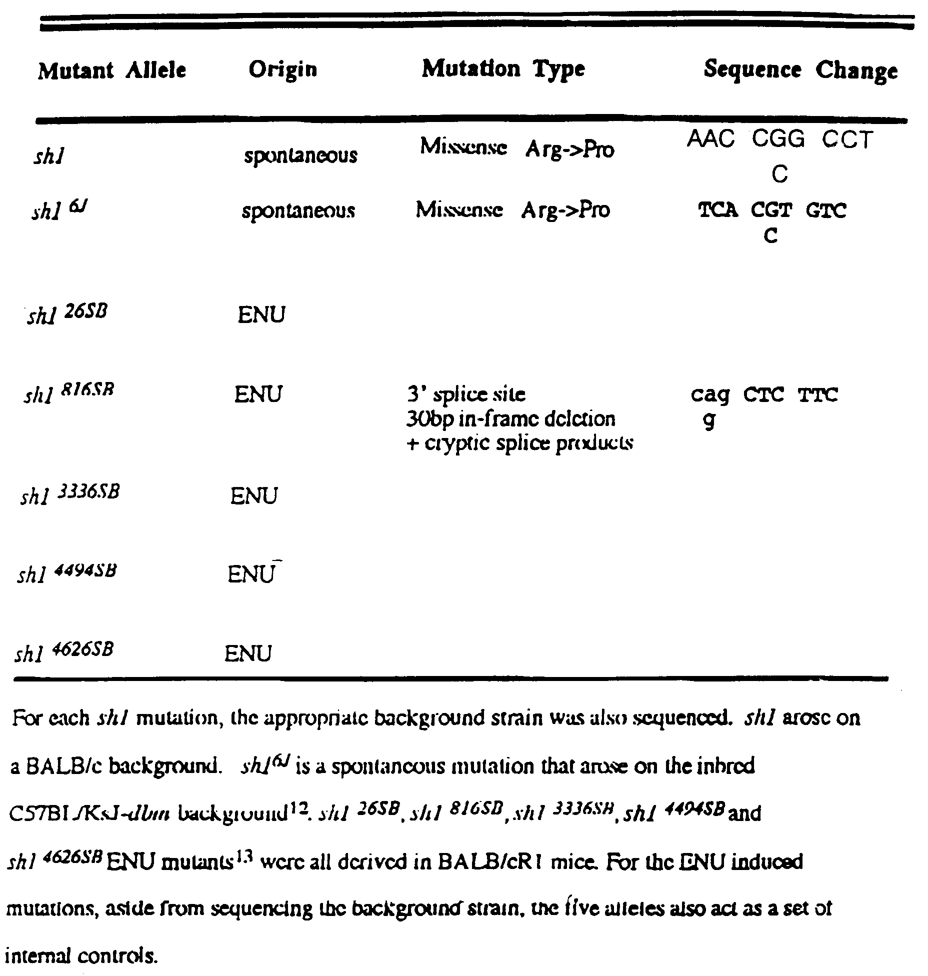

### REFERENCES

1. Usher, C.R. *Lond. Ophtalmol. Hosp. Rep.* **19,** 130-236 (1913/14).
2. SMith, R.J.H., et al., *Am. J. Med. Genet.* **50,** 32-38 (1994).
3. Larget-Piet, D. et al., *Genomics* **21,** 138-143 (1994).
4. Barrong, S.D. et al. *Arch. Ophtalmol.* **110,** 706-710 (1982).
5. Hunter, D.G., Fishman, G.A., Metha, R.S. & Kretzer, F.L., *Arch. Ophtalmol.* **104,** 385-389 (1986).
6. Bonneau D. et al., *Med. Genet.,* **30,** 253-254 (1993).
7. Arden, G.C. & Fox, B. *Nature **279,** 534-536 (1979).*
8. Shinkawa, H. & Nadol, J.B. *Ann. Otol. Rhinol. Laryngol.,* **95,** 313-318 (1986).
9. Kaplan, J. et al., *Genomics* **14,** 979-987 (1992).
10. Kimberling, W.J., et al. *Genomics* **14**, 988-994 (1992).
11. Smith, R.J.H. et al. *Genomics* **14,** 995-1002 (1992).
12. Evans, K.L. et al. *Hum. Mol. Genet.* **2,** 115-118 (1993).
13. Brown, K.A., Sutcliffe, M.J., Steel, K.P. & Brown, S.D.M., *Genomics* **13,** 189-193 (1992).
14. Guilford P. et al. *Hum. Mol. Genet.* **3,** 989-993 (1994).
15. Goodson H.V. & Spulich J.A*. Proc. Natl. Acad. Sci. USA,* **90,** 659-663.
16. Bement, W.M., Hasson, T., Wirth, J.A., Cheney, R.E. & Mooseker, M.S. *Proc. Natl. Acad. Sci, USA* **91,** 6549-6553 (1994).
17. Rayment, Y. & Holden, H.M. *TIBS* **19,** 99-142 (1994).
18. Griffai et al., *Nucleic Acids Res.* , 1991, **19,** 3887-3891.
19. Matthews et al., *Anal. Biochem.,* 1988, **169,** 1-25.
20. Mercer et al., *Nature* **349,** 709-712 (1991).
21. Montell, C. & Rubin, G.M. *Cell* **52,** 757-772 (1988).
22. Collucio, L.M. & bretcher, A.J., *Cell Biol.* **108**, 495-502 (1989).
23. Orita et al., 1989, *Genomics,* **5**, 874-879.
24. Gillespie, P.G., Wagner, M.C. & Hudspeth, A.J., *Neuron,* **11**, 581-594 (1993).
25. Nathans, J. *Cell,* **78**, 357-360 (1994).
26. Faktorovich, E.G., Steinberg, R.H., Yasumara, D., Mathes, M.T. & LaVall, M.M., *Nature,* **347**, 83-86 (1990).
27. Chaitin, M.H., Shneider, B.G., Hall, M.O. & Papermaster, D.S., *J. Cell Biol.,* **99,** 239-247 (1984).
28. Tilney, L.G., Tilney, S.M. & DeRosier, D.J., *Annu. Rev. cell. Biol.* **8**, 257-274 (1992).
29. Myers et al. (1985) *Nature,* **313**, 495-498.
30. Sambrook et al. (1989) , *Molecular Cloning, A Laboratory Manual (2nd Edition). In Cold Spring Harbor University Press, Cold Spring Harbor.*
31. Lord, E.M. & Gates, W.H., *Am. Naturalist* **63**, 435-442 (1929).
32. Deol, M.S., *proc. Roy. Soc. B* **145**, 206-213 (1956).
33. Kituchi, K. & Hilding, D.A., *Acta Otolaryngol.* **60**, 287-303 (1965).
34. Mikaelian, D.O. & Ruben, R.J. *Arch. Otaloaryngol.* **80**, 418-430 (1964).
35. Steel, K.P. & Bock, G.R., *Arch. Otolaryngol.* **109**, 22629 (1983).
36. Brown, K.A., Sutcliffe, M.J., Steel, K.P. and Borwn, S.D.M., *Genomics* **13**, 189-193 (1992).
37. Gibson, F., Lehrach, H., Buckler,A., Brown S.D.M. & North, M.A. *Biotechniques* **16**, 453-458 (1994).
38. Mooseker, M., *Curr. Biol*., **3**, 245-248 (1993).
39. Bement, W.M., Hasson, T., Wirth, J.A., Cheney, R.E. & Mooseker, M.S. *Proc. Natl. Acad. Sci. USA,* **91,** 6549-6553 (1994).
40. Hudspeth, A.J. and Gillespie, P.G., *Neuron,* **12**, 1-9 (1994).
41. Burke, D.T., Rossi, J.M., Lung, J., Koos, D.S. & Tilghman, S.M.*, Mamm. Genome,* **1**, 65 (1991).
42. Chartier, F.L. et al., *Nature Genetics,* **1**, 132-136 (1992).
43. Kusumi, K., Smith, J.S., Segre, J.A., Koos, D.S. & Lander, E.S., *Mamm. Genome,* **4,** 391-392.
44. Hermanson, G.G., Hockstra, M.F., McElligot, D.L. & Evans, G.A., *Nucl. Acids res.,* **19**, 4943-4948.
45. Greenfield, A.J. & Brown, S.D.M., *Genomics,* **1, 153-158 (1987).**

## Claims

1. DNA comprising all or part of a nucleotide sequence selected from SEQ ID N° 1, sequences hybridizing under stringent conditions to all or part of SEQ ID N° 1 and any sequence which code for the same aminoacid sequence as encoded by the nucleotide sequence SEQ ID N° 1 due to the degeneracy of the genetic code.

2. DNA according to claim 1 consisting of the cDNA of sequence SEQ ID N° 1.

3. DNA according to claim 1, consisting of genomic DNA of human USH1B and DFNB2 genes or of murine sh1 gene.

4. DNA according to claim 2 consisting of the cDNA of sequence SEQ ID N° 2.

5. DNA according to claim 1 consisting of the cDNA of sequence SEQ ID N° 3.

6. DNA according to claim 1 consisting of genomic DNA of murine sh1 gene of sequence SEQ ID N°5.

7. DNA consisting of any sequence which hybridizes under stringent conditions to the human gene DFNB2/USHER1B or to the murine gene sh1.

8. Vector containing a DNA according to anyone of claims 1 to 7.

9. Vector according to claim 8 consisting of one of the following plasmids I-1525, I-1526, I-1527, I-1528 or one of the following phages I-1529 and I-1530.

10. DNA insert contained in anyone of the plasmids or phages according to claim 9.

11. Nucleic probe specific for the human USH1B or DFNB2 genes or the murine sh1 gene, comprising at least 20 consecutive nucleotides selected from the nucleotide sequences as defined in anyone of claims 1 to 7.

12. Oligonucleotide primers for the specific amplification of the human sh1 and DFNB2 genes or the murine sh1 gene or of fragments thereof, comprising fragments of nucleotide sequences as defined in anyone of claims 1 to 7.

13. Oligonucleotide primers as defined in claim 12, wherein the sequences have a length of 18 to 30 nucleotides and, preferably, of 18 to 22 nucleotides.

14. Polypeptide or protein encoded by a nucleotide sequence as defined in anyone of claim 1 to 7.

15. Human myosin VII having the peptide sequence SEQ ID N° 3 as well as any derivative thereof having substantially the same functionality as the naturally occuring protein.

16. Protein sequence of the murine myosin VII having the peptide sequence SEQ ID N° 6 as well as a peptide sequence derived from SEQ ID N° 6 as well as any derivative thereof having substantially the same functionality as the naturally occurring protein.

17. Monoclonal or polyclonal antibodies recognizing a protein or a polypeptide as defined in claim 14 to 16.

18. Use of a DNA as defined in anyone of claims 1 to 7, or of a polypeptide sequence as defined in claims 14 to 16, or an antibody as defined in claim 15 for the in vitro diagnostic of non-syndromic or syndromic deafness.

19. Method of detection of a genetic abnormality linked to syndromic or non-syndromic deafness in a biological sample containing human DNA comprising the following steps :
a) bringing the biological sample containing the human DNA into contact with a pair of specific primers according to claim 12 or 13, the human DNA contained in the sample having been optionally rendered accessible to hybridization and under conditions permitting a hybridization of the primers with the human DNA contained in the biological sample;
b) amplification of the human DNA;
c) revealing the amplification products;
d) detecting a genomic abnormality by an appropriate procedure.

20. Method according to claim 19 wherein step d) consists in detecting a mutation of one or more base(s) for example by one of the procedures selected from direct sequencing, SSCP (single-strand conformation polymorphism) analysis, and DGGE (denaturating gradient gel electrophoresis) of the amplification products or fragments thereof.

21. Method according to claim 19 wherein step d) consists in detecting a deletion of one or more bases, for example by direct sequencing of the amplification products or fragments thereof, or RFLP (restriction fragments length polymorphism).

22. Method according to claim 21 for the diagnosis of USHER1B syndrom or of non-syndromic deafness associated with DFNB2.

23. Method of in vitro diagnosis of a human syndromic or non-syndromic deafness consisting in determining a mutation of one or more bases or a deletion of one or more bases of the nucleotide sequence of a human gene coding for an unconventional myosin molecule.

24. Kit for the detection of a genetic abnormality linked to syndromic or non-syndromic deafness in a biological sample comprising :
- at least a pair of oligonucleotides as defined in claim 12 or 13;
- the reagents necessary for carrying out a DNA amplification;
- optionally a component for detecting a mutation or deletion of the amplification products or fragments thereof.

25. Pharmaceutical composition comprising all or part of the nucleic acid of a gene encoding an unconventional myosin molecule.

26. Pharmaceutical composition according to claim 25 comprising a DNA as defined in anyone of claim 1 to 7 or in claim 10.

27. Non human mammalian animal whose germ cells and somatic cells contain a DNA as defined in claim 1 as a result of chromosomal incorporation into the animal genome, or into an ancestor of said animal, of said DNA.

28. Cell lines transfected with a cDNA according to claim 2, 4, 5, or with a genomic DNA according to claim 3.
